# EUROPEAN PATENT APPLICATION

(11) **EP 3 335 692 A1**
(43) Date of publication of application: **20.06.2018**
(21) Application number: 16839352.8
(22) Date of filing: 25.08.2016
(51) Int. Cl.: A61K 8/60, A61K 8/06, A61K 8/31, A61K 8/37, A61K 8/41, A61K 8/49, A61K 8/89, A61K 9/107, A61K 47/14, A61K 47/26, A61Q 19/00, A61K 8/92, A61K 8/04

(54) **METHOD FOR PREPARING HIGH-WATER-CONTENT EMULSION GEL USING OIL GEL-FORMING AGENT**

(30) Priority: 25.08.2015 JP 2015166130
(71) Applicant: Nissan Chemical Industries, Ltd., Chiyoda-ku Tokyo 101-0054 (JP); Kyushu University, Fukuoka-shi, Fukuoka 812-8581 (JP); Institute of Systems, Information Technologies and Nanotechnologies, Fukuoka-shi, Fukuoka 814-0001 (JP)
(72) Inventor: SARUHASHI, Koichiro, Funabashi-shi Chiba 274-0052 (JP); HIRATA, Osamu, Funabashi-shi Chiba 274-0052 (JP); ONO, Fumiyasu, Fukuoka-shi Fukuoka 812-8581 (JP); SHINKAI, Seiji, Fukuoka-shi Fukuoka 811-0201 (JP); YAMAMOTO, Tatsuhiro, Fukuoka-shi Fukuoka 819-0025 (JP)
(74) Representative: Hoffmann Eitle
(86) International application number: PCT/JP2016/074862
(87) International publication number: WO 2017/034005

(57) **Abstract**

There is provided a stable high-water-content W/O-type emulsion gel. A W/O-type emulsion gel comprising: a gelator composed of a compound of Formula (1) or (2) below 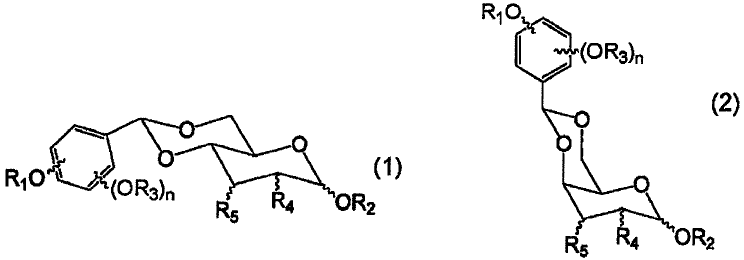 (wherein, R₁ and R₃ are each independently a linear or branched alkyl group having a carbon atom number of 1 to 20, a cyclic alkyl group having a carbon atom number of 3 to 20, or a linear or branched alkenyl group having a carbon atom number of 2 to 20, n is an integer of 0 or 1 to 4, R₂ is a hydrogen atom, a linear or branched alkyl group having a carbon atom number of 1 to 10, or an aryl group optionally having a substituent, and R₄ and R₅ are a hydroxyl group); a surfactant; and a mixed solvent of a hydrophobic organic solvent and water, wherein the content of the gelator is 0.1-20% by mass with respect to the mass of the mixed solvent.

## Description

### TECHNICAL FIELD

The present invention relates to a high-water-content emulsion gel using an oil gelling agent containing a saccharide derivative, and particularly, to a high-water-content W/O-type emulsion gel and a method for preparing the same.

### BACKGROUND ART

A structure containing a fluid in a three-dimensional network structure formed by a substance (hereinafter referred to as a gelator) having a gel-forming ability is called a gel. Generally, a gel is called a hydrogel when the fluid is water, and is called an organogel or oil gel when the fluid is an organic liquid other than water (such as an organic solvent or oil). Oil gels (organogels) are used to adjust the fluidity of cosmetics and paints in the field of cosmetics, pharmaceuticals, pesticides, foods, adhesives, paints, resins, and the like. Oil gels are also widely used in the field of environmental conservation, such as, for example, the prevention of water contamination by gelating waste oil into solid matter.

Furthermore, recently, a low molecular weight gelator has recently been reported which uses a saccharide derivative and is capable of achieving gelation of a mixed solvent of water and oil (hydrophobic solvent) (Patent Document 1).

In addition, water/oil-mixed emulsions are used in the field of cosmetics and the like. Generally, oil-in-water-type (O/W-type) emulsions exhibit better spreadability on skin and have a less oily feeling than water-in-oil-type (W/O-type) emulsions, and thus have more applications in cosmetics. However, W/O-type emulsions also provide a refreshing feeling when the water content in a dispersed phase (internal phase) increases. Thus, it is known that W/O-type emulsions are applied to products that are required to both provide refreshing feeling and exhibit water resistance (Non-Patent Document 1).

It is also known that W/O-type emulsions exhibit skin moistness or cosmetic effects that last for a long time, and that silicone-based W/O-type emulsions not only give a soft and smooth feeling, but are also less likely to cause perspiration-induced deterioration of cosmetic effects (Non-Patent Document 2).

### Prior-Art Documents

### Patent Documents

Patent Document 1: International Publication No. 2013-056493

### Non-Patent Document

Non-Patent Document 1: J. Soc. Chem. Jpn., Vol. 4, No. 2, 103-117 (2010)
Non-Patent Document 2: "New Perspectives on the Molecular Chemistry of Interfaces", March 2011, edited by The Chemical Society of Japan

### SUMMARY OF THE INVENTION

### Problems to be Solved by the Invention

However, in the field of cosmetics, no report has been made on the application of a W/O-type emulsion or silicone-based W/O-type emulsion gel having a high water content in a dispersed phase (internal phase), i.e., a high-water-content W/O-type emulsion or silicone-based W/O-type emulsion gel.

In addition, Patent Document 1 indicates that an emulsion gel can be formed from a mixed solvent containing water and oil (hydrophobic organic solvent), but do not disclose a high-water-content W/O-type emulsion gel. It is generally known that when a high-water-content mixed solvent of water and oil is used as a gel, an O/W-type emulsion gel is easily obtained, however, a W/O-type emulsion is unstable, even if it is obtained.

Therefore, in the field of cosmetics and the like, there is a demand for a stable high-water-content W/O-type emulsion gel which exhibits excellent spreadability on skin and is remarkable in terms of both providing a refreshing feeling and exhibiting water resistance.

The present invention has been made based on the above findings, and the purpose of the present invention is to provide a stable high-water-content W/O-type emulsion gel obtained from a mixed solvent of water and oil.

### Means for Solving the Problems

As a result of diligent research to solve the above problem, the present inventors found that the combination of a surfactant and a low molecular weight gelator using a saccharide derivative can form a W/O-type emulsion gel even in a high-water-content mixed solvent of water and oil (hydrophobic organic solvent), and were thus able to complete the present invention.

That is, according to a first aspect, the present invention pertains to a W/O-type emulsion gel comprising:
a gelator composed of a compound of Formula (1) or (2) below 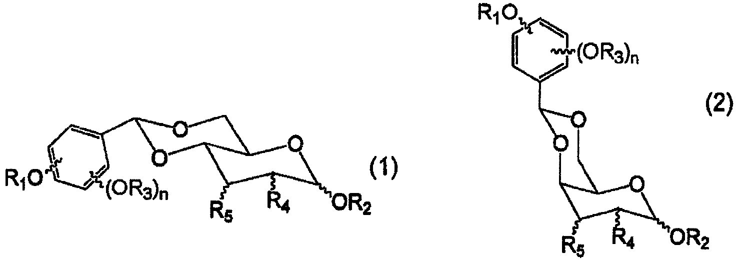
(wherein, R₁ and R₃ are each independently a linear or branched alkyl group having a carbon atom number of 1 to 20, a cyclic alkyl group having a carbon atom number of 3 to 20, or a linear or branched alkenyl group having a carbon atom number of 2 to 20, n is an integer of 0 or 1 to 4,
R₂ is a hydrogen atom, a linear or branched alkyl group having a carbon atom number of 1 to 10, or an aryl group optionally having a substituent, and
R₄ and R₅ are a hydroxyl group);
a surfactant; and
a mixed solvent of a hydrophobic organic solvent and water,
wherein the content of the gelator is 1 to 10% by mass with respect to the mass of the mixed solvent.

According to a second aspect, the present invention pertains to a gel according to the first aspect, wherein the surfactant is at least one surfactant selected from among a nonionic surfactant, an anionic surfactant, or a cationic surfactant, the nonionic surfactant being selected from the group consisting of sorbitan monolaurate, sorbitan monostearate, sorbitan trioleate, polyoxyethylene sorbitan monolaurate, and polyoxyethylene sorbitan monooleate, the anionic surfactant being selected from the group consisting of sodium lauryl sulfate and di(2-ethylhexyl) sodium sulfosuccinate (AOT), and the cationic surfactant being selected from the group consisting of hexadecyltrimethylammonium bromide and cetylpyridinium chloride.

According to a third aspect, the present invention pertains to a gel according to the first or second aspect, wherein the compound of Formula (1) is a compound of Formula (7)
below 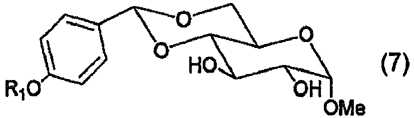 (wherein, R₁ is as defined in Formula (1), and Me is a methyl group).

According to a fourth aspect, the present invention pertains to a gel according to any one of the first to third aspects, wherein the hydrophobic organic solvent is at least one selected from the group consisting of vegetable oils, esters, silicone oils, and hydrocarbons.

According to a fifth aspect, the present invention pertains to a cosmetic or pharmaceutical base material containing the gel according to any one of the first to fourth aspects.

According to a sixth aspect, the present invention pertains to a cosmetic or pharmaceutical base material containing the gel according to any one of the first to fourth aspects and at least one polymeric compound.

According to a seventh aspect, the present invention pertains to method for preparing a W/O-type emulsion gel, the method comprising:
a step in which
a hydrophobic organic solvent and water,
0.1-20% by mass of a gelator with respect to the total mass of the organic solvent and the water, the gelator being composed of a compound of Formula (1) or (2) below 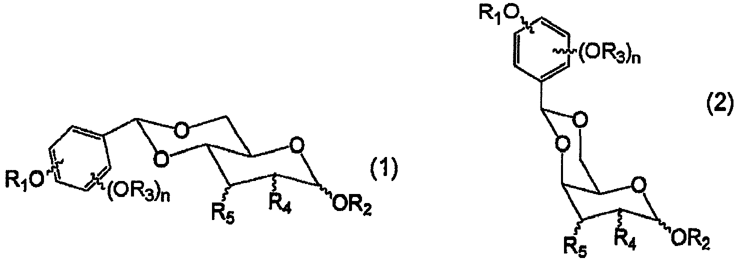
(wherein, R₁ and R₃ are each independently a linear or branched alkyl group having a carbon atom number of 1 to 20, a cyclic alkyl group having a carbon atom number of 3 to 20, or a linear or branched alkenyl group having a carbon atom number of 2 to 20, n is an integer of 0 or 1 to 4,
R₂ is a hydrogen atom, a linear or branched alkyl group having a carbon atom number of 1 to 10, or an aryl group optionally having a substituent, and
R₄ and R₅ are a hydroxyl group), and
a surfactant are mixed, and the resultant mixture is dissolved via heating; and a step for cooling the resultant solution.

### Effects of the Invention

The present invention can provide a W/O-type emulsion gel even in a water/oil (hydrophobic organic solvent) system having a high water content. In addition, the present invention uses a monosaccharide, such as glucose or a derivative thereof, as a raw material of a gelator, and can thus provide a gel which exhibits excellent biological safety and can be used in the field of cosmetics and the like.

Since the W/O-type emulsion gel of the present invention has an oily continuous phase and an aqueous dispersed phase, the W/O-type emulsion gel is not easily washed off with sweat or water, and can exhibit excellent spreadability on skin and provide a refreshing feeling.

In addition, the W/O-type emulsion gel of the present invention has good storage stability.

### BRIEF DESCRIPTION OF THE DRAWINGS

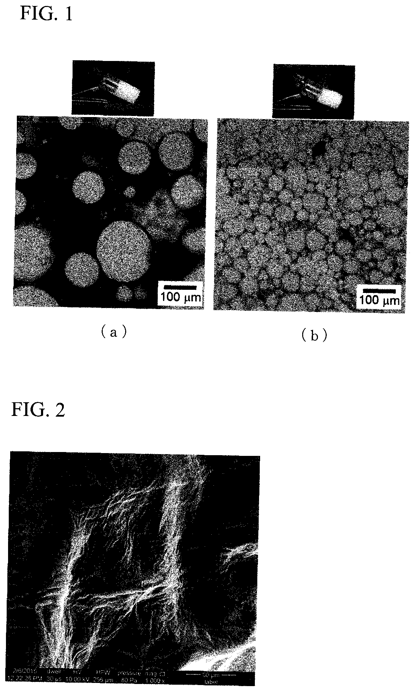

[FIG. 1] FIG. 1 (a) shows a photograph of the external appearance of a water/KF995 dispersion gel (water/KF995: 5/5 (vol/vol)) using glucose derivative [3] as described in Example 3, and a confocal laser scanning microscope image of the dispersion gel. FIG. 1 (b) shows a photograph of the external appearance of a water/KF995 dispersion gel (water/KF995: 8/2 (vol/vol)) in which Tween 20 is added as described in Example 4, and a confocal laser scanning microscope image of the dispersion gel.
[FIG. 2] FIG. 2 shows an SEM image of a 1% Tween 20-containing aqueous solution-gel using glucose derivative [3] as described in Example 4.

### MODES FOR CARRYING OUT THE INVENTION

### [Gelator]

The gelator used in the gel of the present invention is composed of a compound of Formula (1) or (2) below 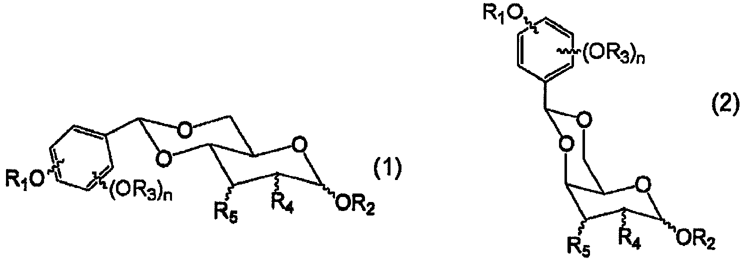
(wherein, R₁ and R₃ are each independently a linear or branched alkyl group having a carbon atom number of 1 to 20, a cyclic alkyl group having a carbon atom number of 3 to 20, or a linear or branched alkenyl group having a carbon atom number of 2 to 20, n is an integer of 0 or 1 to 4,
R₂ is a hydrogen atom, a linear or branched alkyl group having a carbon atom number of 1 to 10, or an aryl group optionally having a substituent, and
R₄ and R₅ are a hydroxyl group).

Examples of the linear alkyl group having a carbon atom number of 1 to 20 include a methyl group, an ethyl group, an n-propyl group, an n-butyl group, an n-pentyl group, an n-hexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group, an n-eicosyl group, and the like.

Examples of the branched alkyl group having a carbon atom number of 1 to 20 include an isopropyl group, an isobutyl group, a sec-butyl group, a tert-butyl group, a 2-ethylhexyl group, and the like.

Examples of the cyclic alkyl group having a carbon atom number of 3 to 20 include a group having a cyclopentyl ring structure, a cyclohexyl ring structure, or the like.

Examples of the linear alkenyl group having a carbon atom number of 2 to 20 include a vinyl group, an allyl group, a butenyl group, a pentenyl group, a hexenyl group, a hepteny group, an octenyl group, a nonenyl group, a decenyl group, and the like.

Examples of the branched alkenyl group having a carbon atom number of 2 to 20 include a 2-methyl-2-propenyl group, an isopropenyl group, a 2-methyl-1-propenyl group, a 2-methylallyl group, and the like.

Examples of the linear or branched alkyl group having a carbon atom number of 1 to 10 include those having a carbon atom number of 1 to 10 among the linear alkyl group having a carbon atom number of 1 to 20 and branched alkyl group having a carbon atom number of 1 to 20 mentioned above.

Examples of the aryl group include a phenyl group, a benzyl group, a 1-naphthyl group, a 2-naphthyl group, a 1-anthryl group, a 1-phenanthryl group, and the like. In addition, the aryl group may have a substituent, and examples of such a substituent include a halogen atom, a linear, branched, or cyclic alkyl group which may contain an ester bond, an amide bond or an ether bond, and the like.

From the viewpoint of using a gelator to achieve favorable gelation of a solvent, R₂ in Formula (1) or (2) is preferably a hydrogen atom or a methyl group.

In addition, from the viewpoint of being able to form a highly transparent gel without syneresis when used in a high-water-content mixed solvent of water and oil (hydrophobic organic solvent), R1 is preferably an octyl group, a decyl group, or a dodecyl group.

A compound of Formula (1) or (2) is obtained by a known method, for example, by reacting a monosaccharide with benzaldehyde dimethyl acetal having the substituent on a benzene ring.

Although the use of a monosaccharide is not particularly limited as long as the monosaccharide has a pyranose ring structure, examples thereof include allose, altrose, glucose, mannose, gulose, idose, galactose, talose, and the like.

Among these, from the viewpoint of being relatively inexpensive and being expected to have especially high biocompatibility, glucose is preferable as the monosaccharide.

Among the compounds of Formula (1) or (2), a compound of Formula (3) below, which has a glucose moiety, is particularly preferable 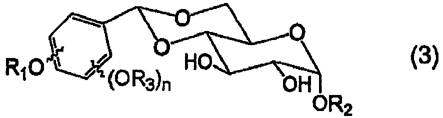 (wherein, R₁, R₂, R₃, and n are as defined in Formula (1)).

Among the compounds of Formula (3), a compound of Formula (5) below is preferable, and a compound of Formula (7) is more preferable. 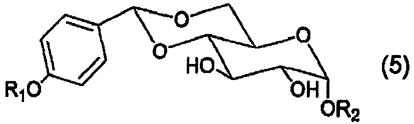 (In Formula (5), R₁ and R₂ are as defined in Formula (1).) 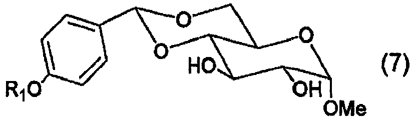 (In Formula (7), R₁ is as defined in Formula (1), and Me is a methyl group.)

For example, R₁ in the above formulae is preferably an alkyl group having a carbon atom number of 8 to 12, particularly an alkyl group having a carbon atom number of 12.

In particular, a compound of Formula (3) (glucose-type gelator) has, as its greatest feature, the feature of exhibiting a gel-forming ability in both water and oil (hydrophobic organic solvent), and is capable of forming a gel in a water/oil mixed solvent. In addition, the compound enables an alcohol-based solvent to be gelled, and forms a highly transparent gel in water.

### [Gel]

The W/O-type emulsion gel of the present invention can be obtained by gelating the gelator, a surfactant, and a mixed solvent of a hydrophobic organic solvent and water. Specifically, a preparation method in which a predetermined amount of the gelator is dissolved via heating in a mixed solvent and the resultant solution is cooled is illustrated. Typically, it is preferable to completely dissolve the gelator during the dissolution via heating.

In addition, the term "gelation" herein refers to a state in which a liquid having fluidity loses the fluidity.

Although not particularly limited as long as the effects of the present invention are exhibited, the amount of the gelator used in the present invention is 0.1-20% by mass, and preferably 0.2-10% by mass, for example, 0.25-5% by mass, with respect to the mass of the mixed solvent.

The aforementioned gelator may be added to a mixed solvent, dissolved via heating with stirring if necessary, and then left at room temperature to obtain a gel. The gel strength can be adjusted by varying the concentration of the gelator.

The surfactant may be any one that, in combination with the gelator, enables a W/O-type emulsion gel to be obtained from a mixed solvent of a hydrophobic organic solvent and water, and a surfactant having lower toxicity, particularly a non-toxic one, is preferable. For example, a nonionic surfactant, an anionic surfactant, and a cationic surfactant may be preferable. Examples of the nonionic surfactant include sorbitan monolaurate, sorbitan monostearate, sorbitan trioleate, polyoxyethylene sorbitan monolaurate, polyoxyethylene sorbitan monooleate, and the like, examples of the anionic surfactant include sodium lauryl sulfate (sodium dodecyl sulfate), sodium di(2-ethylhexyl) sulfosuccinate (sodium bis(2-ethylhexyl) sulfosuccinate) (AOT), and the like, examples of the cationic surfactant include hexadecyltrimethylammonium bromide (hexadecyltrimethylammonium bromide), cetylpyridinium chloride (hexadecylpyridinium chloride-hydrate), and the like, and these may be used alone or in combinations of two or more.

In addition, the surfactant is not limited to a surfactant having a low HLB value, which used as an emulsifier for conventional W/O-type emulsions, and a surfactant having a high HLB value, which is used an emulsifier for O/W-type emulsions, may be used.

For example, polyoxyethylene sorbitan monolaurate (Tween 20; having a HLB value of 16.7), known as an emulsifier for O/W-type emulsions, is preferable.

The amount of the surfactant used in the present invention is not particularly limited as long as the effects of the present invention are exhibited, and can be appropriately selected according to the types of gelator and surfactant used. However, for example, the amount of the surfactant is 0.1-20% by mass, preferably 0.5 to 10% by mass, with respect to the mass of the mixed solvent.

In the present invention, a more uniform and stable W/O-type emulsion gel, which has a dispersed phase (water droplet) having a small particle size, can be obtained by the addition of a surfactant.

Although the solvent is not particularly limited as long as gelation is not prevented, preferred specific examples of the solvent include a mixed solvent of a hydrophobic organic solvent and water, and the like. The ratio between a hydrophobic organic solvent and water may be appropriately selected according to a desired gel. For example, the volume ratio between a hydrophobic organic solvent and water is 5:95 to 95:5, preferably 70:30 to 5:95. The term "high-water-content" herein means that the proportion of water is highest among the components of the gel, for example, the proportion of water in the gel is 50 vol% or more, for example, 75 vol% or more.

Preferred specific examples of the hydrophobic organic solvent include vegetable oils such as olive oil, coconut oil, castor oil, jojoba oil, or sunflower oil; esters such as cetyl octanoate, isopropyl myristate, or isopropyl palmitate; and hydrocarbons such as toluene, xylene, n-hexane, cyclohexane, octane, mineral oil, silicone oil, or hydrogenated polyisobutene.

Among these, olive oil, isopropyl myristate, toluene, cyclohexane, silicon oils such as a linear silicone, a cyclic silicone, an alkyl-modified silicone, a phenyl-modified silicone, dimethicone, or dimethiconol, and octane are preferable as the hydrophobic organic solvent.

A linear silicone (trade name: 2-1184), a cyclic silicone (trade name: SH 245), an alkyl-modified silicone (trade name: SS-3408), a phenyl-modified silicone (trade name: PH-1555), dimethicone (trade name: BY-11-0 series), dimethiconol (trade name: CB-1556), and the like, which are available from Dow Corning Toray Co., Ltd., may be used as the silicone oil. A cyclic silicone (trade name: KF-995) available from Shin-Etsu Chemical Co., Ltd. may also be used.

In a range in which the gel-forming ability of the gelator is not inhibited, various additives (organic compounds such as ultraviolet absorbers, moisturizers, antiseptics, antioxidants, perfumes, or physiologically active substances (active ingredient); inorganic compounds such as titanium oxide, talc, mica, or water; and the like) may also be added, as necessary, to the gel of the present invention depending on the application of the gel and the like.

### [Cosmetic or pharmaceutical base material]

The cosmetic or pharmaceutical base material of the present invention contains the above mentioned gel.

The cosmetic or pharmaceutical base material of the present invention may also contain an alcohol, a polyhydric alcohol, a hydrophilic organic solvent, or a mixed solvent thereof, in addition to the gelator, the nonionic activator, the water, and the hydrophobic organic solvent.

The hydrophilic organic solvent refers to an organic solvent which dissolves in water to an arbitrary percentage, and examples thereof include alcohol, acetone, cyclohexanone, acetonitrile, dioxane, glycerol, dimethylsulfoxide, and the like.

The alcohol is preferably a water-soluble alcohol which freely dissolves in water, more preferably an alcohol having a carbon atom number of 1 to 9, a polyhydric alcohol, a higher alcohol, and glycerides.

Specifically, examples of the alcohol having a carbon atom number of 1 to 9 include methanol, ethanol, 2-propanol, i-butanol, pentanol, hexanol, 1-octanol, isooctanol, and the like; examples of the polyhydric alcohol include ethylene glycol, propylene glycol, polypropylene glycol, and the like; examples of the higher alcohol include octyldodecanol, stearyl alcohol, oleyl alcohol, and the like; and examples of glycerides include trioctanoin, tri(capryl caprylic acid) glyceryl, glyceryl stearate, and the like.

Among these, as the hydrophilic organic solvent, methanol, ethanol, 2-propanol, i-butanol, pentanol, hexanol, 1-octanol, isooctanol, acetone, cyclohexanone, acetonitrile, dioxane, glycerol, propylene glycol, ethylene glycol, and dimethylsulfoxide are preferable, and glycerol, propylene glycol, and ethylene glycol are more preferable.

The cosmetic or pharmaceutical base material of the present invention may also contain, as necessary, additives such as physiologically active substances and functional substances, which are generally incorporated in cosmetic or pharmaceutical base materials, and examples of such additives include oily bases, humectants, feel enhancers, surfactants, polymers, thickening/gelling agents, solvents, propellants, antioxidants, reducing agents, oxidizing agents, preservatives, antibacterial agents, bactericides, chelating agents, pH adjusting agents, acids, alkalis, powders, inorganic salts, ultraviolet absorbers, whitening agents, vitamins and derivatives thereof, hair growth promoters, blood circulation promoters, stimulants, hormones, anti-wrinkle agents, anti-aging agents, tightening agents, cooling agents, warming agents, wound healing promoters, irritation reducing agents, analgesic agents, cell activators, plant/animal/microbial extracts, antipruritic agents, desquamating/keratolytic agents, antiperspirants, fresheners, astringents, enzymes, nucleic acids, perfumes, colorants, coloring agents, dyes, pigments, anti-inflammatory agents, antiphlogistils, antiasthmatic agents, anti-chronic obstructive pulmonary disease drugs, anti-allergic agents, immunomodulators, anti-infective agents, antifungal agents, and the like.

The cosmetic or pharmaceutical base material of the present invention also contains the gelator and at least one polymeric compound.

Examples of the polymeric compound include gelatin, sodium alginate, propylene glycol alginate, gum arabic, polyvinyl alcohol, polyacrylic acid, sodium polyacrylate, carboxymethyl cellulose, gellan gum, Xanthan gum, carrageenan polystyrene, polymethyl methacrylate, polyvinyl pyrrolidone, polyethylene oxide, polylactic acid, polystyrene sulfonic acid, polyacrylonitrile, polyethylene, polyethylene terephthalate, and the like.

In addition, the gel of the present invention can be used for materials in various fields, such as cell culture substrates, substrates for storing biomolecules such as cells and proteins, substrates for external use, biochemical substrates, food bases, contact lenses, disposable diapers, artificial actuators, and arid agricultural substrates. The gel of the present invention can also be widely used as a bioreactor carrier such as an enzyme in various industries such as research, healthcare, and analysis.

### [Method for preparing gelator]

The method for preparing the gelator used in the gel of the present invention is not particularly limited. However, for example, a step, in which a compound of Formula [A] below is reacted with an acetalizing agent, and then the resultant acetal derivative is subjected to a condensation reaction with glucose or a derivative thereof to prepare a compound of Formula (1) or (2), may be performed in one-pot operation in the presence of ethanol and p-toluenesulfonic acid, 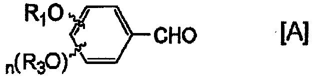 (wherein, R₁ and R₃ are each independently a linear or branched alkyl group having a carbon atom number of 1 to 20, a cyclic alkyl group having a carbon atom number of 3 to 20, or a linear or branched alkenyl group having a carbon atom number of 2 to 20, and n is an integer of 0 or 1 to 4).

### [Examples]

Hereinafter, in order to further clarify the features of the present invention, the following examples are described. However, the present invention is not limited to these examples.

Reagents used as raw materials for synthesis in the examples are shown below.

P-(dodecyloxy) benzaldehyde was obtained from Wako Pure Chemical Industries, Ltd., and methyl α-D-glucopyranoside, p-toluenesulfonic acid monohydrate, and triethyl orthoformate were obtained from Tokyo Chemical Industry Co., Ltd.

N,N-dimethylformamide (DMF) (dehydrated, for organic synthesis) used as a reaction solvent was obtained from Wako Pure Chemical Industries, Ltd.

Sodium bicarbonate (special grade) used in post-reaction treatment and purification was obtained from Wako Pure Chemical Industries, Ltd., and hexane (special grade) was obtained from Kanto Chemical Co., Ltd.

Deuterochloroform (containing 0.03% TMS (tetramethylsilane)) used for NMR measurement was obtained from Sigma-Aldrich Japan Co., Ltd.

Solvents and reagents used in gelation tests and emulsion preparation are shown below.

Polyoxyethylene (20) sorbitan monolaurate (equivalent to Tween 20), isopropyl myristate (first grade), octane (special grade), and sodium lauryl sulfate (for biochemistry) were obtained from Wako Pure Chemical Industries, hexadecylpyridinium chloride monohydrate, hexadecyltrimethylammonium bromide, sodium bis(2-ethylhexyl) sulfosuccinate, and Span 80 (sorbitan monooleate) were obtained from Tokyo Chemical Industry Co., Ltd., and KF-995 was obtained from Shin-Etsu Chemical Co., Ltd. The water used was pure water.

The apparatus and conditions used for various measurements, analysis and polymerization are shown below.
(1) ¹H-NMR spectrum
   - Apparatus: AVANCE 500, manufactured by Bruker BioSpin K.K. JNM-ECS 400, manufactured by JEOL Ltd.
(2) Vortex mixer
   - Apparatus: Voltex Genie 2, manufactured by Scientific Industries, Inc.
(3) Confocal laser scanning microscope
   - Apparatus: LSM 700, manufactured by Carl Zeiss AG
(4) Scanning electron microscope (SEM)
   - Apparatus: Inspect S50, manufactured by FEI Company

### [Example 1: Synthesis of saccharide derivative]

### <Synthesis of glucose derivative>

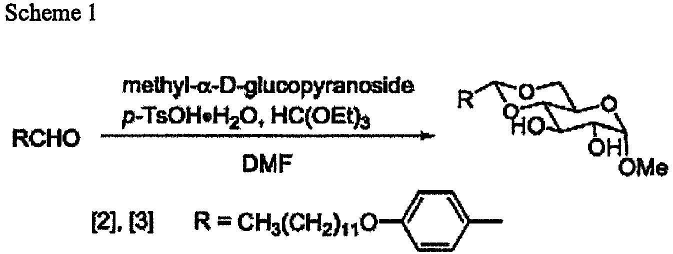

Synthesis of compound [3]: Triethyl orthoformate (5.0 mL, 30 mmol) was added to a DMF (100 mL) suspension of methyl-α-D-glucopyranoside (8.74 g, 45 mmol), p-(dodecyloxy) benzaldehyde [2] (8.71 g, 30 mmol) and p-toluenesulfonic acid monohydrate (143 mg, 0.75 mmol) at room temperature. The reaction solution was heated to 50°C and depressurized. After 6 hours, the reaction solution was allowed to cool to room temperature, 200 mL of saturated aqueous sodium hydrogen carbonate solution was added, and the mixture was filtered. The residue on the filter paper was washed with 400 mL of pure water, and to the obtained solid was added pure water and heated at 90°C for 30 minutes. The resulting suspension solution was filtered and washed with 400 mL of pure water. The obtained solid was dried under reduced pressure, finely pulverized, and then subjected to solid-liquid separation and washing with 300 mL of hexane at room temperature. The suspension was filtered and washed with 400 mL of hexane, and then the white powder was dried under reduced pressure to obtain the target compound.
Yield: 83% (11.7 g) ¹H NMR (400MHz, CDCl₃): *δ* 7.39 (2H, d, *J* = 8.7 Hz), 6.88 (2H, d, *J* = 8.7 Hz), 5.48 (1H, s), 4.80 (1H, d, *J* = 4.1 Hz), 4.27 (1H, dd, *J* = 4.6,9.6 Hz), 3.98-3.88 (3H, m), 3.84-3.68 (2H, m), 3.63 (1H, dt, *J* = 4.1, 9.2 Hz), 3.52-3.41 (4H, m), 2.72 (1H, s), 2.27 (1H, d, *J* = 9.6 Hz), 1.76 (2H, quintet, *J* = 6.9 Hz), 1.50-1.16 (18H, m), 0.88 (3H, t, *J* = 6.9 Hz).

### Example 2: [Gelation test]

### <Gelation test performed adding surfactant>

If a gel can be formed even when a surfactant is added, it follows that a water/oil dispersion gel can be easily prepared. Therefore, the gel-forming ability when Tween 20 (HLB=16.7) having a high HLB value was added was investigated.

The gelation test performed adding a surfactant was carried out as follows. 0.5 wt% surfactant-containing aqueous solutions were prepared, in which various surfactants (polyoxyethylene (20) sorbitan monolaurate (Tween 20) which is a nonionic surfactant, hexadecylpyridinium chloride monohydrate (CPC) and hexadecyltrimethylammonium bromide (CTAB), which are cationic surfactants, and sodium dodecyl sulfate (SDS) and sodium bis(2-ethylhexyl) sulfosuccinate (AOT), which are anionic surfactants) were dissolved in water. Subsequently, a gelator (glucose derivative [3]) was added, to a predetermined concentration, to a 4 mL screw tube, and the prepared surfactant-containing aqueous solution was added thereto and heated at 100°C for 30 minutes. The obtained solution was cooled to room temperature and left for one day. The formation of gel was checked by turning the screw tube upside down. The results are shown in Table 1.

**Table 1 Results of the gelation test for 0.5 wt% surfactant-containing aqueous solutions**

| Gelator | [3] |
|---|---|
| 0.5 wt% Tween 20-containing aqueous solution | 0.25TL |
| 0.5 wt% CPC-containing aqueous solution | 0.25TL |
| 0.5 wt% CTAB-containing aqueous solution | 0.25TL |
| 0.5 wt% SDS-containing aqueous | 0.1 TP |
| 0.5 wt% AOT-containing aqueous solution | 0.25TL |
| Water | Not dissolved |

| | |
|---|---|
| * The numerical values in the table indicate the minimum concentration (wt%) of the gelator (compound) required to gelate each solvent. * TL: translucent gel. TP: transparent gel. | |

As shown in Table 1, the obtained results indicate that a hydrogel can be formed by adding glucose derivative [3] and Tween 20 as a surfactant, etc. to water. On the other hand, the glucose derivative [3] alone cannot form a hydrogel. The feature in which a hydrogel can be formed by the addition of an oil gelling agent and a surfactant (in particular, the hydrogel can be formed regardless of the type of surfactant) is a very distinct example.

In addition, by the same method, a hydrogel having self-sustainable hardness (self-supporting property) was obtained from an aqueous solution containing 2 wt% of glucose derivative [3] and 1 wt% of Tween 20.

### [Example 3: Water/oil dispersion test using glucose derivative]

### <Preparation test of water/KF-995 dispersion gel>

An emulsion preparation technology for uniformly dispersing and stabilizing an oil agent and water is required for various applications, such as cosmetics, pharmaceuticals, foods, functional materials, and the like.

Suppressing the coalescence of droplets by gelating a continuous phase is an important factor in the stabilization of an emulsion. In this experimental result, the W/O-type emulsion gel of the present invention has the potential to stabilize an emulsion by inducing gelation of a continuous phase in W/O-type and O/W-type emulsions.

Therefore, in Example 3, a water/oil dispersion test was conducted in order to verify whether the W/O-type emulsion gel of the present invention can uniformly disperse both solvents of water and an oil agent.

The water/oil dispersion test was carried out as follows. A gelator (glucose derivative [3]), an oil agent (KF-995), and water were added, to predetermined concentrations, to a 4 mL screw-capped sample tube. When Tween 20 was used in the addition of a surfactant, a 1 wt% Tween 20-containing aqueous solution was used instead of water. The sample tube, which contains a mixed solvent containing the gelator and the like, was dissolved via heating for 30 minutes. Subsequently, the sample was sheared off using a vortex mixer, and then left for 1 hour at room temperature to observe the dispersed state. A state in which the solution lost fluidity after cooling so that the solution did not flow down even when the sample tube was turned upside down, and in which water and oil were uniformly dispersed, was determined to be a "water/oil dispersion gel". The obtained results are shown in Table 2.

**Table 2 Preparation test of water/KF-995 dispersion gel using glucose derivative [3]**

| Glucose derivative | | [3] | |
|---|---|---|---|
| Additive | | None | Tween 20 |
| KF-995/water (vol/vol) | 7/3 | 1 (100,3 min.) | |
| | 5/5 | 0.25 (100°C, 3 min.) | |
| | 3/7 | 2 (100°C, 3 min.) | 1 (100°C, 2 min.) |
| | 2/8 | | 2 (100°C, 3 min.) |

| | | | |
|---|---|---|---|
| * The numerical values in the table indicate the concentration (wt%) of the gelator (compound) required to gelate each solvent. * The numerical values in parentheses in the table indicate heating temperature (°C) and shearing time (min.) by vortex mixer. * Diagonal lines indicate that the test was not conducted. | | | |

As shown in Table 2, even when the gelator (glucose derivative [3]) was used alone in the preparation of a water/KF-995 dispersion gel, the dispersion gel could be prepared at a ratio of water/KF-995 in which the fraction of water was larger than that of KF-995, as well as a ratio of water/KF-995 in which the fraction of KF-995 (oil agent) was larger than that of water. In addition, the dispersion gel could be prepared even when a surfactant wad added.

Here, it was found that when 0.05 wt% of the gelator (glucose derivative [3]) was added and the ratio between 1 wt% Tween 20-containing aqueous solution and KF995 was 30/70 (vol/vol), the test conducted under the same preparation conditions as above produced an O/W-type emulsion instead of a gel.

### <Preparation test of water/octane and water/isopropyl myristate (IPM) dispersion gels>

Based on the water/KF-995 dispersion gel preparation conditions, the preparation of a dispersion gel was investigated using an aqueous solution containing 2 wt% of glucose derivative [3] and 1 wt% of Tween 20 when the ratio of water/octane and the ratio of water/IPM were 80/20 (vol/vol). The results are shown in Table 3.

**Table 3 Preparation test of water/octane and water/IPM dispersion gels using glucose derivative [3]**

| Solvent | Water/octane | Water/IPM |
|---|---|---|
| Gelation | Yes (100°C, 5 min.) | Yes (100°C, 5 min.) |

| | | |
|---|---|---|
| * The numerical values in parentheses in the table indicate heating temperature (°C) and shearing time (min.) by vortex mixer. | | |

In addition, based on the water/KF-995 dispersion gel preparation conditions, the preparation of a dispersion gel was investigated using 0.5 wt% or 1.0 wt% of glucose derivative [3] and 0.5 wt% of Span 80 when the ratio of water/octane was 80/20, 85/15, 90/10 and 95/5 (vol/vol). The results are shown in Table 4.

**Table 4 Preparation test of water-octane dispersion gel using glucose derivative [3]**

| Water-octane ratio (vol/vol) | 80/20 | 85/15 | 90/10 | 95/5 |
|---|---|---|---|---|
| Gelator concentration (wt%) | 1.0 (100°C, 2 min.) | 0.5 (100°C, 5min.) | 0.5 (100°C, 5min.) | 0.5 (100°C, 5min.) |

| | | | | |
|---|---|---|---|---|
| * The numerical values in parentheses in the table indicate heating temperature (°C) and shearing time (min.) by vortex mixer. | | | | |

From the results in tables 2, 3, and 4, it was revealed that a gel emulsion having a high internal phase ratio in various solvents can be prepared with a gelator concentration of 2 wt% or less.

### <Identification of dispersed phase and continuous phase in dispersion gel using confocal laser microscope>

An emulsion gel was prepared in the same manner as above using an aqueous solution of 5 µM uranine, which is a fluorescent dye, instead of water, and confocal laser scanning microscope was used to identify whether water is locally present in a dispersed phase or locally present in a continuous phase. A photograph of the obtained sample tube and a microscope image of the emulsion gel are shown in FIG. 1.

From FIG. 1 (a), it was found that the water/KF-995 dispersion gel (water/KF-995: 5/5 (vol/vol)) using the glucose derivative [3] is a W/O-type emulsion gel (having an aqueous dispersed phase and a continuous phase of KF-995). From FIG. 1 (b), it was found that the high-water-content water/KF-995 dispersion gel (water/KF-995: 8/2 (vol/vol)) prepared using the 1 wt% Tween 20-containing aqueous solution instead of water is a W/O-type emulsion gel (having an aqueous dispersed phase and a continuous phase of KF995).

Generally, Tween 20 is a surfactant used for forming an O/W-type emulsion. Meanwhile, in this experimental result, a W/O-type emulsion gel was formed. From the results in Example 2, it was found that the glucose derivative [3], which does not have a gel-forming ability in water, can be made to form a hydrogel by adding Tween 20 to water. From these facts, it is suggested that a complex of glucose derivative [3] and Tween 20 is involved in the formation of an emulsion gel.

In addition, in cosmetic applications, high-water-content W/O-type emulsions are not easily washed off with sweat or water, exhibit excellent spreadability on skin, and are expected to be applied to products that are required to both feel refreshing and exhibit water resistance. However, high-water-content W/O-type emulsions are known to be generally unstable. By using the method according to the present invention, it was shown that a high-water-content W/O-type emulsion, which is considered to be difficult to prepare, can be prepared as a gel.

### [Example 4: SEM observation of gel]

### <SEM observation of 1% Tween 20-containing aqueous solution-gel (hydrogel) using glucose derivative [3]>

SEM observation of 1% Tween 20-containing aqueous solution-gel (hydrogel) was carried out as follows. A 1% Tween 20-containing aqueous solution-gel, in which 2 wt% of glucose derivative [3] was added, was prepared, and then was placed on a carbon tape and observed using SEM under a reduced pressure of 80 Pa. The obtained result is shown in FIG. 2.

As shown in the SEM image in FIG. 2, a phase in which fibrous structures were bundled was obtained from the 1% Tween 20-containing aqueous solution-gel (hydrogel) using glucose derivative [3]. From this fact, it was revealed that the hydrogel composed of glucose derivative [3] and surfactant Tween 20 formed a gel based on a fibrous structure.

## Claims

1. A W/O-type emulsion gel comprising:
a gelator composed of a compound of Formula (1) or (2) below 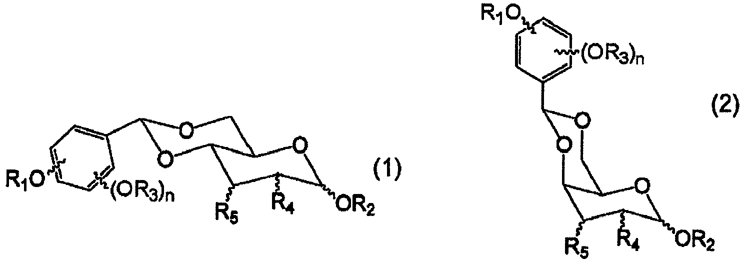
(wherein, R₁ and R₃ are each independently a linear or branched alkyl group having a carbon atom number of 1 to 20, a cyclic alkyl group having a carbon atom number of 3 to 20, or a linear or branched alkenyl group having a carbon atom number of 2 to 20, n is an integer of 0 or 1 to 4,
R₂ is a hydrogen atom, a linear or branched alkyl group having a carbon atom number of 1 to 10, or an aryl group optionally having a substituent, and
R₄ and R₅ are a hydroxyl group);
a surfactant; and
a mixed solvent of a hydrophobic organic solvent and water,
wherein the content of the gelator is 0.1-20% by mass with respect to the mass of the mixed solvent.

2. The gel according to claim 1, wherein the surfactant is at least one surfactant selected from among a nonionic surfactant, an anionic surfactant, or a cationic surfactant, the nonionic surfactant being selected from the group consisting of sorbitan monolaurate, sorbitan monostearate, sorbitan trioleate, polyoxyethylene sorbitan monolaurate, and polyoxyethylene sorbitan monooleate, the anionic surfactant being selected from the group consisting of sodium lauryl sulfate and di(2-ethylhexyl) sodium sulfosuccinate (AOT), and the cationic surfactant being selected from the group consisting of hexadecyltrimethylammonium bromide and cetylpyridinium chloride.

3. The gel according to claim 1 or 2, wherein the compound of Formula (1) is a compound of Formula (7) below 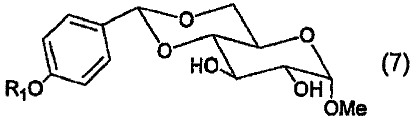 (wherein, R₁ is as defined in Formula (1), and Me is a methyl group).

4. The gel according to any one of claims 1 to 3, wherein the hydrophobic organic solvent is at least one selected from the group consisting of vegetable oils, esters, silicone oils, and hydrocarbons.

5. A cosmetic or pharmaceutical base material containing the gel according to any one of claims 1 to 4.

6. A cosmetic or pharmaceutical base material containing the gel according to any one of claims 1 to 4, and at least one polymeric compound.

7. A method for preparing a W/O-type emulsion gel, the method comprising:
a step in which
a hydrophobic organic solvent and water,
0.1-20% by mass of a gelator with respect to the total mass of the hydrophobic organic solvent and the water, the gelator being composed of a compound of Formula (1) or (2) below 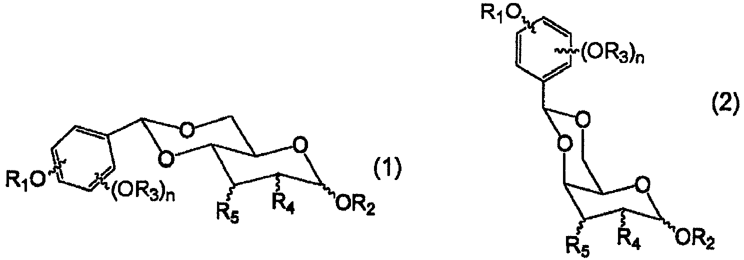
(wherein, R₁ and R₃ are each independently a linear or branched alkyl group having a carbon atom number of 1 to 20, a cyclic alkyl group having a carbon atom number of 3 to 20, or a linear or branched alkenyl group having a carbon atom number of 2 to 20, n is an integer of 0 or 1 to 4,
R₂ is a hydrogen atom, a linear or branched alkyl group having a carbon atom number of 1 to 10, or an aryl group optionally having a substituent, and
R₄ and R₅ are a hydroxyl group), and
a surfactant are mixed, and the resultant mixture is dissolved via heating; and
a step for cooling the resultant solution.
